(19) Europäisches Patentamt
European Patent Office
Office européen des brevets

(11) Veröffentlichungsnummer: **0 387 821 B1**

# (12) EUROPÄISCHE PATENTSCHRIFT

(45) Veröffentlichungstag der Patentschrift: **24.08.94**

(51) Int. Cl.⁵: **C07D 215/42**, A61K 31/475

(21) Anmeldenummer: **90104777.9**

(22) Anmeldetag: **14.03.90**

## (54) 2-Alkyl-4-arylmethylaminochinoline, ihre Verwendung und daraus hergestellte Arzneimittel.

(30) Priorität: **17.03.89 DE 3908767**
**28.12.89 DE 3943158**

(43) Veröffentlichungstag der Anmeldung:
**19.09.90 Patentblatt 90/38**

(45) Bekanntmachung des Hinweises auf die Patenterteilung:
**24.08.94 Patentblatt 94/34**

(84) Benannte Vertragsstaaten:
**AT BE CH DE DK FR GB IT LI NL SE**

(56) Entgegenhaltungen:
**EP-A- 0 258 755**
**EP-A- 0 259 174**
**FR-A- 2 321 291**
**GB-A- 2 047 244**

(73) Patentinhaber: **BASF Aktiengesellschaft**
**Carl-Bosch-Strasse 38**
**D-67063 Ludwigshafen (DE)**

(72) Erfinder: **Geiss, Karl-Heinz, Dr.**
**Eckbachring 46**
**D-6711 Heuchelheim (DE)**
Erfinder: **Ruebsamen, Klaus, Dr.**
**Erschigweg 19**
**D-6730 Neustadt (DE)**
Erfinder: **Traut, Martin, Dr.**
**Muehltalstrasse 125**
**D-6900 Heidelberg (DE)**

EP 0 387 821 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 2-Alkyl-4-arylmethylaminochinoline sowie deren Verwendung bei der Bekämpfung von Krankheiten.

In folgenden Literaturstellen wurden 8-substituierte 4-Benzylamino-2-methylchinoline mit potentiell amöbizider und z.T. fungizider Wirkung beschrieben:
J. Indian Chem. Soc. 51 (1974), 880-882
J. Indian Chem. Soc. 56 (1979), 1265-1268
Ann.Biochem.Exptl.Med. (Calcutta) Suppl.20 (1960) 493-504 (= CA-58, 8254c)
J.scient.ind. Res. India 13B (1954), 15-21
4-Arylmethylaminochinoline mit fungizider Wirksamkeit werden in US 4,744,823 beschrieben.

In allgemeiner Form wurden 2-Alkyl-4-benzylamino-chinoline als Zwischenprodukte in der US-P 3 075 984 genannt; 2,8-Dimethyl-4-benzylaminochinolin wurde in Bull.Soc.Chim. France 1973, 2860-2864 sowie in C.R. Acad. Sci., Ser. C 275 (1972), 1041-1044 beschrieben. Für diese Verbindungen wurden jedoch keine pharmakologischen Wirkungen beschrieben.

Aus dem weiteren Umfeld pharmakologisch aktiver 4-Aminochinoline mit stark variiertem Substitutionsmuster am Chinolingerüst sind zu nennen:
In 2-Position unsubstituierte 4-Anilino- und 4-Phenylalkylaminochinolin-3-yl-ketone und -carbonsäureester mit magensäuresekretionshemmender Wirkung wurden in US-P 4 343 804 sowie in der EP-OS 259 174 beschrieben.

In der EP-OS 258 755 wurden 4-Amino-2-methylchinolin-3-yl-ketone und -alkanole zur Behandlung von Morbus Alzheimer beansprucht.

Es wurde nun überraschenderweise gefunden, daß die erfindungsgemäßen Verbindungen der Formel I

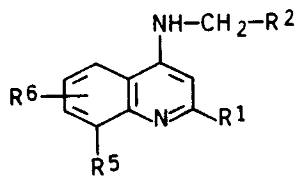

(I)

in der

$R^1$ eine $C_1$-$C_3$-Alkylgruppe, die durch eine Hydroxy- oder eine $C_1$-$C_3$-Alkoxygruppe substituiert sein kann,

$R^2$ entweder einen Naphthylrest oder einen Rest der Formel (a),

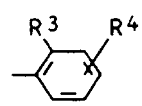

(a)

bedeutet, wobei

$R^3$ eine $C_1$-$C_3$-Alkylgruppe, die durch eine Hydroxy- oder Methoxygruppe substituiert sein kann, eine $C_1$-$C_3$-Alkoxygruppe, ein Fluor-, Chlor- oder Bromatom, und

$R^4$ ein Wasserstoffatom, eine Methyl-, Hydroxy- oder Methoxygruppe, ein Fluor-, Chlor- oder Bromatom und

$R^5$ eine $C_1$-$C_3$-Alkoxy-, eine $C_1$-$C_3$-Alkylgruppe, die durch eine Hydroxy- oder $C_1$-$C_3$-Alkoxygruppe substituiert sein kann, eine Hydroxygruppe, ein Fluor-, Chlor- oder Bromatom und

$R^6$ ein Wasserstoffatom, eine Methyl-, Methoxygruppe, ein Fluor-, Chlor- oder Bromatom bedeutet,

sowie deren physiologisch verträgliche Salze wertvolle pharmakologische Wirkungen besitzen. Insbesondere hemmen sie die $K^+/H^+$-ATPase und die Säuresekretion des Magens.

Bevorzugt bedeutet $R^1$ eine Methylgruppe, die durch eine Hydroxy- oder $C_1$-$C_3$-Alkoxygruppe, insbesondere eine Methoxygruppe substituiert sein kann.

Bevorzugt bedeutet $R^2$ einen 1-Naphthylrest oder einen Rest der Formel (a).

Bevorzugt bedeutet $R^3$ eine $C_1$-$C_3$-Alkylgruppe, insbesondere die Methyl- und Ethylgruppe, eine $C_1$-$C_3$-Alkoxygruppe, insbesondere die Methoxygruppe, ein Chlor- oder ein Bromatom.

$R^4$ bedeutet bevorzugt ein Wasserstoffatom, eine 4-Hydroxygruppe oder eine 6-Methyl-, 6-Methoxygruppe oder ein 6-Fluor- oder 6-Chloratom, insbesondere bedeutet $R^4$ ein Wasserstoffatom oder ein 6-Fluor- oder 6-Chloratom.

Bevorzugte Bedeutungen von $R^5$ sind eine $C_1$-$C_2$-Alkoxygruppe, eine $C_1$-$C_2$-Alkylgruppe, die durch eine Hydroxy- oder Methoxygruppe substituiert sein kann. Besonders bevorzugt steht $R^5$ für eine Methoxy-, eine Methyl- oder Ethylgruppe.

Bevorzugte Bedeutung von $R^6$ ist ein Wasserstoffatom.

Die Herstellung der erfindungsgemäßen Verbindungen erfolgt durch Analogieverfahren in an sich bekannter Weise, indem ein Chinolin der Formel II

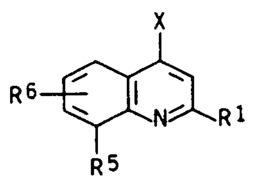

(II),

wobei X eine nukleophile Abgangsgruppe wie ein Chlor- oder Bromatom oder eine Phenoxygruppe darstellt und $R^1$, $R^5$ und $R^6$ die obigen Bedeutungen besitzen, mit einem Amin der Formel $R^2$-$CH_2$-$NH_2$ in üblicher Weise umgesetzt wird.

Die Reaktion kann in Gegenwart eines Lösungsmittels wie Toluol, Xylol, Phenol, Ethanol, Dimethylsulfoxid, Dimethylethylenharnstoff, Dimethylpropylenharnstoff, Pyrrolidon, N-Methylpyrrolidon, in Gemischen dieser Lösungsmittel oder in Abwesenheit von Lösungsmitteln gegebenenfalls in Gegenwart eines Katalysators wie Kupfer- oder Bronzepulver oder Kupfer(I)-chlorid bei Temperaturen von 50 bis 250 °C, gegebenenfalls unter Druck, durchgeführt werden. Die Amine $R^2CH_2NH_2$ können in äquimolekularen Mengen oder im Überschuß eingesetzt werden.

Bevorzugt wird die Umsetzung der Verbindungen der Formel II mit den Aminen $R^2CH_2NH_2$ im Verhältnis 1:1 bis 1:10 in Gegenwart von Phenol bei Temperaturen von 60 bis 160 °C durchgeführt.

Die Herstellung von 4-Aminochinolinen nach obigem Verfahren wurde u.a. in folgenden Literaturstellen beschrieben:

G. Jones, Quinolines, Part I, John Wiley & Sons, London, New York, 1977, S. 547-550 u. dort zit. Lit.; J. Indian Chem. Soc. 51 (1974) 880-882; J.Med.Chem. 14 (1971) 1060-1066; Chim. Therap. 1 (1966) 339-346; Eur.J.Med.Chem. 11 (1976) 561-565.

Die Amine $R^2CH_2NH_2$ sowie die Edukte der Formel II sind literaturbekannt, bzw. kommerziell erhältlich, bzw. können analog bekannten Verbindungen hergestellt werden.

Zur Darstellung von 4-Chlor-, 4-Brom- und 4-Phenoxychinolinen siehe G. Jones (Ed) Quinolines, Part I, John Wiley & Sons, London, 1977: X = Cl: S. 391-398, X = Br: S. 404-406 X = $OC_6H_5$: S. 577-579. 4-Phenoxychinoline können auch bei der Umsetzung der 4-Chlorchinoline mit Aminen $R^2CH_2NH_2$ in Gegenwart von Phenol als Zwischenstufen nachgewiesen werden.

Zur Darstellung der Verbindung II, X = Cl, $R^1$ = $CH_3$, $R^5$ = $OCH_3$, $R^6$ = H (CA-Reg.-Nr. 64 951-58-2) siehe z.B. Coll. Czech. Chem. Com. 20 (1955) 1206-1214; J. Chem. Soc. 1932, 1984-1988.

Die erfindungsgemäßen Verbindungen der Formel I, bei denen $R^1$ eine Hydroxymethyl-gruppe bedeutet, können auch durch Reduktion von Verbindungen der allgemeinen Formel III,

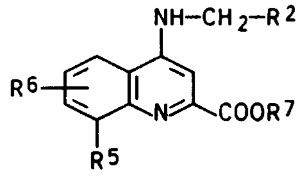

(III),

in der $R^2$, $R^5$ und $R^6$ die für Formel I genannten Bedeutungen besitzt und $R^7$ ein Wasserstoffatom oder eine $C_1$-$C_3$-Alkylgruppe darstellt, mit geeigneten Reduktionsmitteln wie Lithiumaluminiumhydrid erhalten werden.

Die erfindungsgemäßen Verbindungen der Formel I, bei denen $R^1$ eine $C_1$-$C_3$-Alkoxymethylgruppe bedeutet, können auch durch Umsetzung der Verbindungen der Formel IV,

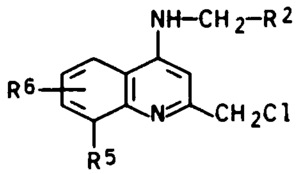

x HCl (IV)

in der $R^2$, $R^5$ und $R^6$ die genannten Bedeutungen besitzen, mit einem Alkali-$C_1$-$C_3$-Alkoxyd in einem Lösungsmittel wie dem entsprechenden $C_1$-$C_3$-Alkohol oder Dimethylformamid erhalten werden.

Die Edukte der Formel IV können durch Umsetzung der Verbindungen der Formel I, in der $R^1$ eine Hydroxymethylgruppe bedeutet, mit Thionylchlorid hergestellt werden. Eine Hydroxygruppe in $R^5$ muß durch eine geeignete Schutzgruppe modifiziert werden, die nach der Umsetzung der Verbindungen IV mit $C_1$-$C_3$-Alkoxiden zu Verbindungen I, $R^1$ = $CH_3O$-$C_1$-$C_3$-Alk wieder abgespalten wird.

Die Edukte der Formel III sind durch Umsetzung der Verbindungen der Formel V,

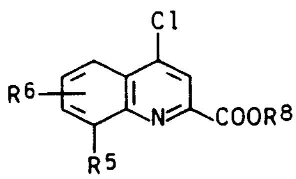

(V)

in der $R^8$ eine $C_1$-$C_4$-Alkylgruppe bedeutet, mit Aminen der Formel $R^2CH_2NH_2$ nach dem für die Umsetzung der Verbindungen (II) mit den Aminen $R^2CH_2NH_2$ beschriebenen Verfahren erhältlich, wobei ggf. die Hydrolyse der Ester (III, $R^7$ = $C_1$-$C_3$-Alkyl) zu den Carbonsäuren (III, $R^7$ = H) angeschlossen werden kann.

Die Edukte der Formel V sind durch Umsetzung der 4-Hydroxychinoline der Formel VI,

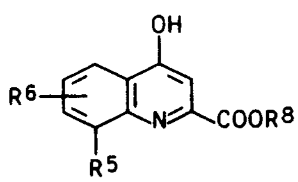

(VI)

in der $R^8$ die für Formel V genannte Bedeutung besitzt, mit üblichen Chlorierungsmitteln wie $POCl_3$, $PCl_5$, $SOCl_2$ erhältlich, wobei eine Hydroxygruppe in $R^5$ zuerst durch eine geeignete Schutzgruppe modifiziert wird, die nach erfolgter Umsetzung zu Verbindungen der Formel (V) wieder abgespalten wird. Zur Darstellung der Verbindungen der Formel VI, $R^6 = H$, s. z.B. J. Org. Chem. 16 (1951) 412-414; J.Amer.Chem.Soc. 73 (1951) 3520; Z. Naturforsch. 35B (1980) 1569-1571; Z. Physiol. Chem. 297 (1954) 247-248.

Gegebenenfalls werden die erhaltenen erfindungsgemäßen Verbindungen in das Säureadditionssalz einer physiologisch verträglichen Säure überführt. Eine Zusammenstellung üblicher physiologisch verträglicher Säuren kann aus Fortschritte der Arzneimittelforschung 1966, Deutschland, Schweiz, Birkhäuser Verlag, Bd. 10. S. 224-285 und J.Pharm.Sci., Bd. 66 (1977), S. 1-5 entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der Aminoverbindungen der Formel I durch Auflösen der freien Basen in einer wäßrigen Säurelösung hergestellt werden.

Die erfindungsgemäßen Verbindungen und ihre Salze mit physiologisch verträglichen Säuren besitzen wertvolle pharmakologische Wirkungen. Insbesondere hemmen sie die gastrointestinale $K^+/H^+$-ATPase und die Magensäuresekretion. Die erfindungsgemäßen Verbindungen können daher zur Therapie aller Krankheiten, bei denen eine Herabsetzung der Magensäuresekretion die Heilung günstig beeinflußt, z.B. Ulcus

ventriculi, Ulcus duodeni, Gastritis, Reflux-Ösophagitis, Zollinger-Ellison-Syndrom eingesetzt werden (vgl. Review über Hemmstoffe der $K^+/H^+$-ATPase, G. Sachs et al., Ann. Rev. Pharmacol. Toxicol. 28 (88) 269-284 u. dort zit. Lit.).

Gegenstand der vorliegenden Erfindung sind auch Arzneimittel zur oralen, rektalen oder intravenösen Applikation, die neben üblichen Träger- und Verdünnungsmitteln die Verbindungen der Formel I oder deren Säureadditionssalze als Wirkstoff enthalten, sowie die Verwendung der neuen Verbindungen und ihrer physiologisch verträglichen Salze bei der Behandlung der genannten Krankheiten.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsmittel wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten, herstellen.

Die Einzeldosierung liegt beim Menschen
für die orale oder rektale Anwendung bei 10 bis 1000 mg pro Einzeldosis, für die i.v. Anwendung bei 0,01 bis 1,0 mg/kg Körpergewicht.

Die Wirkung der erfindungsgemäßen Verbindungen wurde in der folgenden Testanordnung bestimmt:
Die Mukosa eines frisch entnommenen Schweinemagens wird im Eisbad in 0,25 M Saccharose, 20 mM Tris (Trishydroxymethyl-amino-methan), 1 mM EGTA (Ethylenbis(oxyethylennitrilo)-tetraessigsäure) pH 7,0 homogenisiert und 20 min bei 20 000 xg zentrifugiert. Der Überstand wird 60 min bei 100 000 xg zentrifugiert. Das erhaltene mikrosomale Pellet wird mit 50 mM Tris + 2 mM $MgCl_2$ + 0,1 mM EGTA, pH 7,5, homogenisiert und bei -20 °C portionsweise eingefroren. Die $K^+/H^+$-ATPase-Aktivität wird in 1 ml-Ansätzen mit folgender Zusammensetzung getestet: 50 mM Tris/HCl-Puffer, pH 7,5, 2 mM $MgCl_2$, 20 µg Membran-Protein mit oder ohne Zugabe von 5 mM KCl. Die ATPase-Reaktion wird gestartet durch Zugabe von $Na_2ATP$, Endkonzentration 2 mM, Reaktionszeit 15 min bei 37 °C. Danach wird die Reaktion durch Zugabe von 1 ml 20 % Trichloressigsäure gestoppt. Die Bestimmung des freigesetzten Phosphats erfolgt nach der Methode von Sanui (Analyt. Biochem. 60, 1974, 489-504).

Durch Zugabe der erfindungsgemäßen Verbindungen in der obigen Testanordnung wird die $K^+/H^+$-ATPase gehemmt.

Beispiele 1 bis 11, Verbindungen der allgemeinen Formel I, $R^1 = CH_3$, $R^5 = CH_3O$, $R^6 = H$

Allgemeine Arbeitsvorschrift:

Eine Mischung aus 1 Äquivalent 4-Chlor-8-methoxy-2-methylchinolin, 1 bis 11 Äquivalenten eines Amins der Formel $R^2CH_2NH_2$ und 5 bis 20 Äquivalenten Phenol wurde 3 bis 8 Stunden auf 110 bis 140 °C gegebenenfalls im Autoklav erhitzt. Nach Abkühlen der Reaktionsmischung wurde mit Essigester versetzt und mehrmals mit wäßriger Weinsäurelösung extrahiert. Die wäßrige Phase wurde mit konz. $NH_3$ oder mit

verd. NaOH alkalisch gestellt. Dabei kristallisierte ein Teil der Wertprodukte aus, nach Absaugen und Waschen mit Wasser, Ether oder Essigester wurden die Kristalle getrocknet und mit Ether aufgekocht. Nach Absaugen und Trocknen erhielt man die erfindungsgemäßen Verbindungen. Falls aus der alkalischen wäßrigen Phase keine Kristalle ausfielen, wurde mit Essigester mehrmals extrahiert, die org. Phase mehrmals mit verd. NaOH und $H_2O$ gewaschen, über $Na_2SO_4$ getrocknet und am Rotationsverdampfer vom Lösungsmittel befreit. Die Rohprodukte wurden mit Ether aufgekocht und das Produkt abgesaugt und getrocknet. Falls notwendig, wurden die Produkte z.B. aus Isopropanol oder Ethanol umkristallisiert (s. Tabelle).

Nach dieser Vorschrift wurden die Verbindungen der Beispiele 1 bis 11 erhalten.

Tabelle 1: Verbindungen der Formel I, $R^1$ = $CH_3$, $R^5$ = $CH_3O$, $R^6$ = H

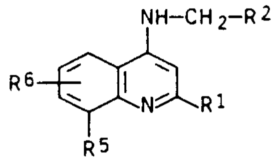

(I)

| Bsp. | $R^2$ | Äquiv. $R^2CH_2NH_2$ | Äquiv. Phenol | Reaktionsbed. (Std) | T (°C) | Ausbeute (%) | Fp. (°C) |
|---|---|---|---|---|---|---|---|
| 1 | 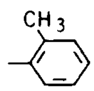 | 11 | 20 | 4 | 130 | 36 | 228-229[a)] |
| 2 | 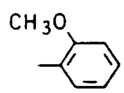 | 11 | 15 | 3 | 130 | 40 | 223-224[b)] |
| 3 | 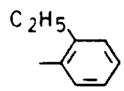 | 1,5 | 5 | 4 | 140 | 69 | 208-209[a)] |
| 4 | 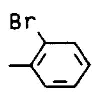 | 1,5 | 15 | 4 | 140 | 88 | 250-251[a)] |
| 5 | 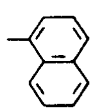 | 1,5 | 15 | 4 | 130 | 75 | 275-276[a)] |
| 6 | 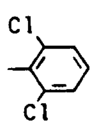 | 1,5 | 15 | 8 | 140 | 84 | 259-260[a)] |
| 7 | 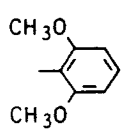 | 1,1 | 11 | 6 | 140 | 31 | 269-270[a)] |
| 8 | 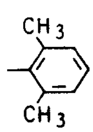 | 1,1 | 11 | 6 | 140 | 11 | 286-287[a)] |

Tabelle 1 (Fortsetzung)

| Bsp. | $R^2$ | Äquiv. $R^2CH_2NH_2$ | Äquiv. Phenol | Reaktionsbed. (Std) | T (°C) | Ausbeute (%) | Fp. (°C) |
|---|---|---|---|---|---|---|---|
| 9 | 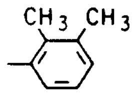 | 1,0 | 11 | 6 | 140 | 27 | 240-241[b)] |
| 10 | 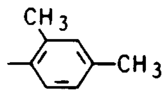 | 1,5 | 15 | 4 | 140 | 51 | 208-209[a)] |
| 11 | 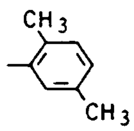 | 1,5 | 15 | 6 | 140 | 64 | 230-231[a)] |

a) aus Ethanol umkristallisiert
b) aus Isopropanol umkristallisiert.

Beispiele 12a-12d, Verbindungen der allgemeinen Formel I, $R^1 = CH_3$, $R^5 = CH_3O$, $R^6 = H$
Analog der allgemeinen Arbeitsvorschrift für die Beispiele 1 bis 11 wurden durch Umsetzung von 4-Chlor-8-methoxy-2-methyl-chinolin mit Aminen $R^2CH_2NH_2$ die Verbindungen 12(a)-12(d) erhalten.

Beispiel 12(a)

4-(2-Chlorbenzylamino)-8-methoxy-2-methyl-chinolin (Schmp.: 230-231 °C)

Beispiel 12(b)

4-(2-Fluorbenzylamino)-8-methoxy-2-methyl-chinolin
Schmp.: 248-249 °C

Beispiel 12(c)

4-(2-Chlor-6-fluorbenzylamino)-8-methoxy-2-methyl-chinolin
Schmp.: 259-260 °C

Beispiel 12(d)

4-(2,6-Difluorbenzylamino)-8-methoxy-2-methyl-chinolin
Schmp.: 258-259 °C

Beispiel 13, Verbindung der allgemeinen Formel I, $R^1 = CH_3$, $R^2 = $ 1-Naphthyl, $R^5 = CH_3O$, $R^6 = H$

a) 8-Methoxy-2-methyl-4-phenoxychinolin
Verbindung der Formel II, $R^1 = CH_3$, $X = OC_6H_5$, $R^5 = CH_3O$, $R^6 = H$
10 g (= 48,2 mMol) 4-Chlor-8-methoxy-2-methylchinolin wurden mit 30 g (= 319 mMol) Phenol in Gegenwart von 80 ml konz. Ammoniaklösung im Autoklav 8 Stunden bei 140 °C gehalten. Der Ansatz wurde mit Essigester verdünnt, die org. Phase mehrmals mit Weinsäurelösung gewaschen. Anschließend wurde die wäßrige Phase mit NaOH auf pH 10 eingestellt und mit Essigester extrahiert. Nach Trocknen der organischen Phase über Natriumsulfat und Abziehen des Lösungsmittels am Rotationsverdampfer wurde der Rückstand mit Ether aufgerührt und das Produkt abgesaugt. Man erhielt 8,4 g = 93 % der obengenannten Verbindung vom Schmp. 140-141 °C.

b) 8-Methoxy-2-methyl-4-(1-naphthylmethylamino)-chinolin
Verbindung der allgemeinen Formel I, $R^1 = CH_3$, $R^2$ = 1-Naphthyl, $R^5 = CH_3O$, $R^6$ = H
2,0 g (= 7,55 mMol) 8-Methoxy-2-methyl-4-phenoxychinolin wurden mit 1,8 g (= 11,5 mMol) 1-Naphthylmethylamin und 7,1 g (= 75 mMol) Phenol im Autoklav 5 Stunden bei 130 °C gehalten. Nach Abkühlen wurde die Reaktionsmischung mit Essigester versetzt und mehrmals mit Weinsäurelösung extrahiert. Die wäßrige Phase wurde mit konz. Ammoniaklösung alkalisch gestellt, das ausgefallene Produkt wurde abgesaugt, mit Ether gewaschen und getrocknet. Man erhielt 1,6 g (= 65 %) 8-Methoxy-2-methyl-4-(1-naphthylmethylamino)-chinolin, das mit der Verbindung aus Beispiel 5 identisch war.

Beispiel 14, Verbindung der allgemeinen Formel I, $R^1 = CH_2OH$, $R^2$ = o-Tolyl, $R^5 = CH_3O$, $R^6$ = H

a) 4-Chlor-8-methoxychinolin-2-carbonsäuremethylester
Verbindung der Formel V, $R^5 = CH_3O$, $R^6$ = H, $R^8 = CH_3$
In 113 ml $POCl_3$ trug man bei Raumtemperatur bis 40 °C portionsweise 57,6 g (247 mMol) 4-Hydroxy-8-methoxychinolincarbonsäuremethylester ein und heizte 2 Stunden bei 80 °C nach. Die dunkle Reaktionsmischung wurde auf Eis gegossen und mit konz. $K_2CO_3$-Lösung auf pH 6 eingestellt. Der ausgefallene Niederschlag wurde abgesaugt und aus DMF/Wasser umkristallisiert. Man erhielt 42,7 g (= 69 %) 4-Chlor-8-methoxy-chinolin-2-carbonsäuremethylester vom Schmp. 139-141 °C.
b) 8-Methoxy-4-(2-methylbenzylamino)-chinolin-2-carbonsäure,
Verbindung der Formel III, $R^2$ = o-Tolyl, $R^5 = CH_3O$, $R^6 = R^7$ = H
Eine Mischung aus 54 g (= 215 mMol) 4-Chlor-8-methoxy-chinolin-2-carbonsäuremethylester, 33,7 g (= 278 mMol) 2-Methylbenzylamin und 243 g (= 2,58 Mol) Phenol wurde im Autoklav 4 Stunden auf 140 °C erhitzt. Die abgekühlte Reaktionsmischung wurde zu 4 l Essigester getropft und der entstandene Niederschlag abgesaugt. Der Festkörper wurde in 1 l THF gelöst und mit 10 % NaOH auf pH 10 eingestellt. Unter Konstanthaltung des pH-Wertes wurde 1 Stunde bei 50 °C gerührt, auf 10 °C abgekühlt und mit verd. HCl auf pH 5-6 eingestellt. Dabei fiel das Produkt kristallin aus. Man erhielt 18,85 g 8-Methoxy-4-(2-methylbenzylamino)-chinolin-2-carbonsäure vom Schmp. 276-277 °C.
c) 2-Hydroxymethyl-8-methoxy-4-(2-methylbenzylamino)-chinolin,
Verbindung der Formel I, $R^1 = CH_2OH$, $R^2$ = o-Tolyl, $R^5 = CH_3O$, $R^6$ = H
Eine Mischung aus 0,50 g (12,9 mMol) $LiAlH_4$, 2,8 g (8,6 mMol) 8-Methoxy-4-(2-methylbenzylamino)-chinolin-2-carbonsäure und 20 ml abs. THF wurde unter $N_2$ 6 Stunden bei Raumtemperatur umgesetzt. Man versetzte nacheinander mit 3,6 ml Essigester, 36 ml Wasser und 17 ml 2 N NaOH. Nach Stehen über Nacht wurde das Filtrat am Rotationsverdampfer eingeengt, mit Wasser versetzt und mit Essigester extrahiert. Nach Trocknen über $Na_2SO_4$ wurde der Essigester abrotiert und der Rückstand in Methanol gelöst und mit methanolischer HCl-Lösung sauer gestellt. Nach Abrotieren des Lösungsmittels am Rotationsverdampfer und Umkristallisation aus Ethanol erhielt man 2,69 g (≙ 81 %) 2-Hydroxymethyl-8-methoxy-4-(2-methylbenzylamino)-chinolin-hydrochlorid vom Schmp. 158-160 °C.

Beispiel 15, Verbindung der Formel I, $R^1 = CH_3OCH_2$, $R^2$ = o-Tolyl, $R^5 = CH_3O$, $R^6$ = H

a) 2-Chlormethyl-8-methoxy-4-(2-methylbenzylamino)-chinolin-hydrochlorid
Verbindung der Formel IV, $R^2$ = o-Tolyl, $R^5 = CH_3O$, $R^6$ = H
Eine Mischung aus 2,5 g (= 7,2 mMol) 2-Hydroxymethyl-8-methoxy-4-(2-methylbenzylamino)-chinolin-hydrochlorid, 5,3 ml Thionylchlorid und 2 Tropfen DMF wurde bei Raumtemperatur 90 Minuten gerührt. Der Ansatz wurde auf Eis gegossen, mit 1 N NaOH alkalisch gestellt und mit Essigester extrahiert. Nach Trocknung der org. Phase mit $Na_2SO_4$ und Abziehen des Lösungsmittels wurde das Rohprodukt mit methanolischer Salzsäure in das Hydrochlorid überführt. Man erhielt 2,48 g 2-Chlormethyl-8-methoxy-4-(2-methylbenzylamino)-chinolin-hydrochlorid.
b) 8-Methoxy-2-methoxymethyl-4-(2-methylbenzylamino)-chinolin
Verbindung der Formel I, $R^1 = CH_3OCH_2$, $R^2$ = o-Tolyl, $R^5 = CH_3O$, $R^6$ = H
800 mg (= 2,2 mMol) 2-Chlormethyl-8-methoxy-4-(2-methylbenzylamino)-chinolin-hydrochlorid wurden in Gegenwart von 1,2 g (= 22 mMol) Natriummethylat in 30 ml absolutem Methanol 5 Stunden bei Rückfluß gerührt. Man engte den Ansatz am Rotationsverdampfer weitgehend ein, versetzte mit Essigester, extrahierte mehrmals mit $K_2CO_3$-Lösung. Nach Trocknen über $Na_2SO_4$ und Entfernung des Lösungsmittels wurde der Rückstand aus Ethanol umkristallisiert. Man erhielt 200 mg (= 28 %) 8-Methoxy-2-methoxymethyl-4-(2-methylbenzylamino)-chinolin vom Schmp.: 194-195 °C.

Beispiel 16, 4-Chlor-8-methoxy-2-methoxymethylchinolin, Formel II, $R^1 = CH_3OCH_2$, $R^5 = CH_3O$, $X = Cl$, $R^6 = H$

a) 3-(2-Methoxyanilino)-4-methoxy-but-2-ensäure-methylester
Eine Mischung aus 100 g o-Anisidin, 190 g γ-Methoxy-acetessigsäuremethylester, 2,3 ml Essigsäure, 203 g Calciumsulfat und 800 ml abs. Ethanol wurde 16 Stunden am Rückfluß gerührt. Nach Filtration und Einengen des Filtrats im Vakuum wurde der Rückstand in Essigester aufgenommen, mehrmals mit 2N NaOH und 5 %iger Citronensäure extrahiert, über $Na_2SO_4$ getrocknet und am Rotationsverdampfer vom Lösungsmittel befreit. Man erhielt 150 g Rohprodukt, das ohne weitere Reinigung in der folgenden Stufe eingesetzt wurde.
b) 4-Hydroxy-8-methoxy-2-methoxymethylchinolin
150 g des Rohprodukts aus Beispiel 16a) wurden in 330 g Polyphosphorsäure bei 80 °C 4 Stunden gerührt. Die Mischung wurde mit 1 l $H_2O$ versetzt und mit conz. NaOH auf pH 6 eingestellt. Durch Extraktion mit Essigester wurde o-Anisidin abgetrennt. Nach Aufsättigen der wäßrigen Phase mit NaCl wurde das Wertprodukt mit $CH_2Cl_2$ extrahiert. Nach Trocknen über $Na_2SO_4$ und Abziehen des Lösungsmittels wurde aus Essigester umkristallisiert. Man erhielt 60 g 4-Hydroxy-8-methoxy-2-methoxymethylchinolin vom Schmp.: 193-195 °C.
c) 4-Chlor-8-methoxy-2-methoxymethylchinolin, Verbindung II, $R^1 = CH_3OCH_2$, $R^5 = CH_3O$, $R^6 = H$, $X = Cl$
60 g der Verbindung aus Beispiel 16b) wurden mit 210 g $POCl_3$ 1 Stunde bei Raumtemperatur und 1 Stunde bei Rückfluß gerührt. Nach dem Abkühlen wurde auf 800 ml Eis gegossen und unter Kühlen mit konz. NaOH alkalisch gestellt. Nach Aufsättigen mit NaCl wurde die wäßrige Phase mit $CH_2Cl_2$ extrahiert. Nach Trocknen über $Na_2SO_4$ und Abrotieren des Lösungsmittels wurde das Rohprodukt durch eine Säulenchromatographie ($SiO_2$, Laufmittel: Essigester) gereinigt. Man erhielt 26 g 4-Chlor-8-methoxy-2-methoxymethylchinolin vom Schmp.: 67-69 °C.

Beispiel 17, Verbindungen der allgemeinen Formel I, $R^1 = CH_3OCH_2$, $R^5 = CH_3O$, $R^6 = H$

Nach der allgemeinen Arbeitsvorschrift für die Beispiele 1 bis 11 wurden durch Umsetzung der Verbindung aus Beispiel 16c) mit Aminen $R^2CH_2NH_2$ und Phenol bei 120 bis 130 °C die Verbindungen der Beispiele 17a) bis 17e) erhalten.

Beispiel 17(a)

8-Methoxy-2-methoxymethyl-4-(2-methylbenzylamino)-chinolin,
Schmp.: 194-195 °C

Beispiel 17(b)

4-(2-Brombenzylamino)-8-methoxy-2-methoxymethyl-chinolin
Schmp.: 202-203 °C

Beispiel 17(c)

4-(2-Chlorbenzylamino)-8-methoxy-2-methoxymethyl-chinolin
Schmp.: 213-214 °C

Beispiel 17(d)

8-Methoxy-4-(2-methoxybenzylamino)-2-methoxymethyl-chinolin
Schmp.: 207-208 °C

Beispiel 17(e)

8-Methoxy-2-methoxymethyl-4-(1-naphthylmethylamino)-chinolin
Schmp.: 211-212 °C
Analog der allgemeinen Arbeitsvorschrift für die Beispiele 1 bis 11 können durch Umsetzung der entsprechenden Verbindungen der allgemeinen Formel II mit den Aminen $R^2CH_2NH_2$ die folgenden Beispiele erhalten werden.

Beispiel 18, Verbindungen der allgemeinen Formel I,

$R^2$= 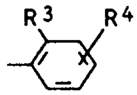 , $R^5=OCH_3$, $R^6=H$

| Bsp. | $R^1$ | $R^3$ | $R^4$ | Fp [°C] |
|---|---|---|---|---|
| 18a | $CH_3$ | $CF_3$ | H | 126-128 |
| 18b | $CH_3$ | $iC_3H_7$ | H | |
| 18c | $C_2H_5$ | Br | H | |
| 18d | $C_2H_5$ | $CH_3$ | H | |
| 18e | $C_2H_5$ | $OCH_3$ | H | |
| 18f | $nC_3H_7$ | Cl | H | |
| 18g | $nC_3H_7$ | Cl | 6-F | |
| 18h | $iC_3H_7$ | $CH_3$ | H | |
| 18i | $iC_3H_7$ | Br | H | |
| 18j | $CH_2OH$ | Br | H | |
| 18k | $CH_2OH$ | Cl | H | |
| 18l | $CH_2OH$ | Cl | 6-Cl | |
| 18m | $CH_2OH$ | $OCH_3$ | H | |
| 18n | $CH_2OC_2H_5$ | $CH_3$ | H | |
| 18o | $CH_2OC_2H_5$ | Br | H | |
| 18p | $CH_2OC_2H_5$ | Cl | H | |
| 18q | $CH_2OiC_3H_7$ | $OCH_3$ | H | |

Beispiel 19, Verbindungen der allgemeinen Formel I,

$R^1=CH_3$, $R^2=$ 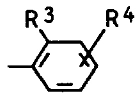 , $R^5=CH_3O$, $R^6=H$

| Bsp. | $R^3$ | $R^4$ | Fp [°C] |
|---|---|---|---|
| 19a | $CH_3$ | 4-OH | |
| 19b | Br | 4-OH | |
| 19c | Cl | 4-OH | |
| 19d | Br | 4-F | 226-227 |
| 19e | $nC_3H_7$ | H | |
| 19f | $CH_2OH$ | H | |
| 19g | $CH_2OCH_3$ | H | |
| 19h | $OC_2H_5$ | H | |
| 19i | $O-nC_3H_7$ | H | |
| 19j | $O-iC_3H_7$ | H | |
| 19k | $OCH_3$ | 6-F | |
| 19l | $OCH_3$ | 6-Cl | |

Beispiel 20, Verbindungen der allgemeinen Formel I

$R^1=CH_3$, $R^2=$ 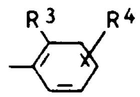 , $R^6=H$

| Bsp. | $R^3$ | $R^4$ | $R^5$ |
|---|---|---|---|
| 20a | $CH_3$ | H | $OC_2H_5$ |
| 20b | Br | H | $OC_2H_5$ |
| 20c | $OCH_3$ | H | $OC_2H_5$ |
| 20d | Cl | H | $OC_2H_5$ |
| 20e | Cl | 6-F | $OC_2H_5$ |
| 20f | Br | H | $O\text{-}nC_3H_7$ |
| 20g | $CH_3$ | H | $O\text{-}nC_3H_7$ |
| 20h | $CH_3$ | H | $CH_2OH$ |
| 20i | Br | H | $CH_2OH$ |
| 20j | Cl | H | $CH_2OH$ |
| 20k | $OCH_3$ | H | $CH_2OH$ |
| 20l | $CH_3$ | H | $CH_2OCH_3$ |
| 20m | Br | H | $CH_2OCH_3$ |
| 20n | Cl | H | $CH_2OCH_3$ |
| 20o | $OCH_3$ | H | $CH_2OCH_3$ |
| 20p | $CH_3$ | H | $CH_2OC_2H_5$ |
| 20q | Br | H | $CH_2OiC_3H_7$ |
| 20r | $CH_3$ | H | OH |
| 20s | Br | H | OH |
| 20t | Cl | H | OH |
| 20u | $OCH_3$ | H | OH |
| 20v | Br | 4-OH | OH |
| 20w | $CH_3$ | H | F |
| 20x | $OCH_3$ | H | F |
| 20y | Br | H | F |
| 20z | Cl | H | F |
| 2a | $CH_3$ | H | Cl |
| 2b | $OCH_3$ | H | Cl |
| 2c | Br | H | Cl |
| 2d | Cl | H | Cl |

| Bsp. | $R^3$ | $R^4$ | $R^5$ |
|---|---|---|---|
| 2e | $OCH_3$ | H | Br |
| 2f | $CH_3$ | H | Br |
| 2g | Br | H | Br |
| 2h | Cl | H | Br |

Beispiel 21, Verbindungen der allgemeinen Formel I,

$R^1=R^5=CH_3$ , $R^2=$ 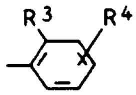 , $R^6=H$

| Bsp. | $R^3$ | $R^4$ |
|---|---|---|
| 21a | $CH_3$ | H |
| 21b | $CH_3$ | 6-$CH_3$ |
| 21c | Cl | 6-Cl |
| 21d | Cl | 6-F |
| 21e | Cl | H |
| 21f | Br | H |
| 21g | Br | 6-Cl |
| 21h | $C_2H_5$ | H |
| 21i | $CH_2OH$ | H |
| 21j | $CH_2OCH_3$ | H |
| 21k | $OCH_3$ | H |
| 21l | $OCH_3$ | 6-$OCH_3$ |
| 21m | $OCH_3$ | 6-Cl |
| 21n | $OC_2H_5$ | H |
| 21o | F | 6-F |

Beispiel 22, Verbindungen der allgemeinen Formel I,

$R^2 =$ 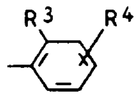 , $R^4 = R^6 = H$

| Bsp. | $R^1$ | $R^3$ | $R^5$ |
|---|---|---|---|
| 22a | $CH_2OH$ | $CH_3$ | $CH_3$ |
| 22b | $CH_2OCH_3$ | $CH_3$ | $CH_3$ |
| 22c | $CH_2OCH_3$ | Br | $CH_3$ |
| 22d | $CH_2OCH_3$ | $OCH_3$ | $CH_3$ |
| 22e | $CH_2OCH_3$ | Cl | $CH_3$ |
| 22f | $CH_2OH$ | Cl | $C_2H_5$ |
| 22g | $CH_2OCH_3$ | Br | $C_2H_5$ |
| 22h | $CH_3$ | $CH_3$ | $C_2H_5$ |
| 22i | $CH_3$ | Br | $C_2H_5$ |
| 22j | $CH_3$ | Cl | $C_2H_5$ |
| 22k | $CH_3$ | $OCH_3$ | $C_2H_5$ |
| 22l | $CH_3$ | $CH_3$ | $i-C_3H_7$ |
| 22m | $CH_3$ | Br | $n-C_3H_7$ |
| 22n | $CH_3$ | $OCH_3$ | $n-C_3H_7$ |

Beispiel 23, Verbindungen der allgemeinen Formel I,

$R^1=CH_3$, $R^2=$ 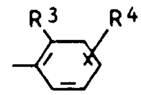 , $R^4=H$, $R^5=OCH_3$

| Bsp. | $R^3$ | $R^6$ |
|---|---|---|
| 23a | $CH_3$ | 6-$OCH_3$ |
| 23b | Br | 6-$OCH_3$ |
| 23c | Cl | 6-$OCH_3$ |
| 23d | $OCH_3$ | 6-$OCH_3$ |
| 23e | $CH_3$ | 5-$OCH_3$ |
| 23f | Br | 5-$OCH_3$ |
| 23g | Cl | 5-$OCH_3$ |
| 23h | $CH_3$ | 5-$CH_3$ |
| 23i | $OCH_3$ | 5-$CH_3$ |
| 23j | Br | 5-$CH_3$ |
| 23k | $CH_3$ | 7-Br |
| 23l | Br | 7-Br |
| 23m | $CH_3$ | 7-Cl |
| 23n | $CH_3$ | 5-Cl |
| 23o | $OCH_3$ | 5-Cl |
| 23p | Br | 5-Cl |
| 23q | Br | 5-Br |
| 23r | Cl | 5-Br |
| 23s | $OCH_3$ | 5-Br |
| 23t | $CH_3$ | 5-Br |

## Patentansprüche

**1.** 2-Alkyl-4-arylmethylaminochinoline der allgemeinen Formel I

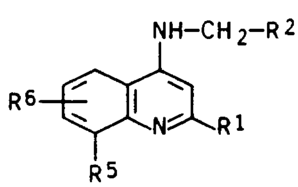

(I)

in der

$R^1$ eine $C_1$-$C_3$-Alkylgruppe, die durch eine Hydroxy- oder eine $C_1$-$C_3$-Alkoxygruppe substituiert sein kann,

$R^2$ entweder einen Naphthylrest oder einen Rest der Formel (a),

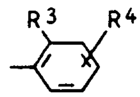

(a)

bedeutet, wobei

$R^3$ eine $C_1$-$C_3$-Alkylgruppe, die durch eine Hydroxy- oder Methoxygruppe substituiert sein kann, eine $C_1$-$C_3$-Alkoxygruppe, ein Fluor-, Chlor- oder Bromatom, und

$R^4$ ein Wasserstoffatom, eine Methyl-, Hydroxy- oder Methoxygruppe, ein Fluor-, Chlor- oder Bromatom, und

$R^5$ eine $C_1$-$C_3$-Alkoxy-, eine $C_1$-$C_3$-Alkylgruppe, die durch eine Hydroxy- oder $C_1$-$C_3$-Alkoxygruppe substituiert sein kann, eine Hydroxygruppe, ein Fluor-, Chlor- oder Bromatom, und

$R^6$ ein Wasserstoffatom, eine Methyl-, Methoxygruppe, ein Fluor-, Chlor- oder Bromatom bedeutet,

sowie deren physiologisch verträgliche Salze.

2. Verbindung gemäß Anspruch 1, worin $R^5$ eine $C_1$-$C_2$-Alkoxygruppe, eine $C_1$-$C_2$-Alkylgruppe, die durch eine Hydroxy- oder Methoxygruppe substituiert sein kann, bedeutet, sowie deren physiologisch verträgliche Salze.

3. Verbindung gemäß Anspruch 2, worin $R^1$ eine Methylgruppe, die durch eine Hydroxy- oder $C_1$-$C_3$-Alkoxygruppe substituiert sein kann, $R^2$ einen 1-Naphthylrest oder eine Gruppe der Formel (a) bedeutet, wobei $R^3$ eine $C_1$-$C_3$-Alkyl-, eine $C_1$-$C_3$-Alkoxygruppe, ein Chlor- oder Bromatom bedeutet und $R^4$ ein Wasserstoffatom, eine 4-Hydroxygruppe, eine 6-Methyl-, 6-Methoxygruppe, ein 6-Fluor- oder 6-Chloratom und $R^6$ ein Wasserstoffatom bedeutet, sowie deren physiologisch verträgliche Salze.

4. Verbindung gemäß Anspruch 3, worin $R^1$ eine Methyl-, Methoxymethyl- oder Hydroxymethylgruppe, $R^2$ einen 1-Naphthylrest oder die Gruppe (a) bedeutet, worin $R^3$ eine Methyl-, Ethyl-, Methoxygruppe, ein Chlor- oder Bromatom bedeutet und $R^4$ ein Wasserstoffatom oder ein 6-Fluor- oder 6-Chloratom, $R^5$ eine Methoxy-, eine Methyl- oder Ethylgruppe und $R^6$ ein Wasserstoffatom bedeutet, sowie deren physiologisch verträgliche Salze.

5. Verfahren zur Herstellung der Verbindungen der Formel I

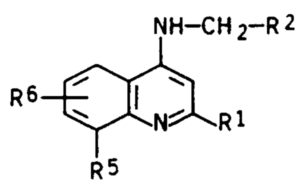

(I)

in der

$R^1$ eine $C_1$-$C_3$-Alkylgruppe, die durch eine Hydroxy- oder eine $C_1$-$C_3$-Alkoxygruppe substituiert sein kann,

$R^2$ entweder einen Naphthylrest oder einen Rest der Formel (a),

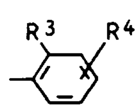

(a)

bedeutet, wobei

$R^3$ eine $C_1$-$C_3$-Alkylgruppe, die durch eine Hydroxy- oder Methoxygruppe substituiert sein kann, eine $C_1$-$C_3$-Alkoxygruppe, ein Fluor-, Chlor- oder Bromatom, und

$R^4$ ein Wasserstoffatom, eine Methyl-, Hydroxy- oder Methoxygruppe, ein Fluor-, Chlor- oder

Bromatom, und

$R^5$ eine $C_1$-$C_3$-Alkoxy-, eine $C_1$-$C_3$-Alkylgruppe, die durch eine Hydroxy- oder $C_1$-$C_3$-Alkoxygruppe substituiert sein kann, eine Hydroxygruppe, ein Fluor-, Chlor- oder Bromatom, und

$R^6$ ein Wasserstoffatom, eine Methyl-, Methoxygruppe, ein Chlor- oder Bromatom bedeutet,

dadurch gekennzeichnet, daß ein Chinolin der Formel II

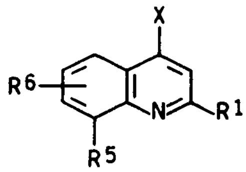

(II),

wobei X eine nukleophile Abgangsgruppe darstellt und $R^1$, $R^5$ und $R^6$ die im Anspruch 1 genannten Bedeutungen besitzen, mit einem Amin der Formel $R^2CH_2NH_2$ in Gegenwart oder Abwesenheit eines Lösungsmittels bei einer Temperatur im Bereich von 50 bis 250 °C umgesetzt wird.

**6.** Verwendung einer Verbindung der allgemeinen Formel I nach einem der Ansprüche 1 bis 4 zur Herstellung eines Arzneimittels.

**7.** Verwendung einer Verbindung der allgemeinen Formel I nach einem der Ansprüche 1 bis 4 zur Herstellung eines Arzneimittels gegen Krankheiten, bei denen eine Herabsetzung der Magensäuresekretion die Heilung günstig beeinflußt.

**8.** Arzneimittel für die orale oder rektale Anwendung, das als Wirkstoff pro Einzeldosis 10 bis 1000 mg einer Verbindung der Formel I nach einem der Ansprüche 1 bis 4 neben üblichen galenischen Hilfsmitteln enthält.

**9.** Arzneimittel für die intravenöse Applikation, das als Wirkstoff 0,01 bis 1 mg/kg Körpergewicht einer Verbindung der Formel I nach einem der Ansprüche 1 bis 4 neben üblichen galenischen Hilfsmitteln enthält.

**10.** Arzneimittel gegen Krankheiten, bei denen eine Herabsetzung der Magensäuresekretion die Heilung günstig beeinflußt, das als Wirkstoff eine wirksame Menge einer Verbindung der Formel I nach einem der Ansprüche 1 bis 4 neben üblichen galenischen Hilfsmitteln enthält.

## Claims

**1.** A 2-alkyl-4-arylmethylaminoquinoline of the formula I

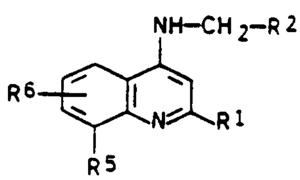

(I)

where

$R^1$ is $C_1$-$C_3$-alkyl which can be substituted by a hydroxyl or $C_1$-$C_3$-alkoxy group,

$R^2$ is either naphthyl or a radical of the formula (a)

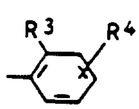

(a)

where

$R^3$ is $C_1$-$C_3$-alkyl which can be substituted by a hydroxyl or methoxy group, or is $C_1$-$C_3$-alkoxy, fluorine, chlorine or bromine, and

$R^4$ is hydrogen, methyl, hydroxyl, methoxy, fluorine, chlorine or bromine, and

$R^5$ is $C_1$-$C_3$-alkoxy, $C_1$-$C_3$-alkyl which can be substituted by a hydroxyl or $C_1$-$C_3$-alkoxy group, or is hydroxyl, fluorine, chlorine or bromine, and

$R^6$ is hydrogen, methyl, methoxy, fluorine, chlorine or bromine,

and the physiologically tolerated salts thereof.

**2.** A compound as claimed in claim 1, where $R^5$ is $C_1$-$C_2$-alkoxy, or $C_1$-$C_2$-alkyl which can be substituted by a hydroxyl or methoxy group, and the physiologically tolerated salts thereof.

**3.** A compound as claimed in claim 2, where $R^1$ is methyl which can be substituted by a hydroxyl or $C_1$-$C_3$-alkoxy group, $R^2$ is 1-naphthyl or a group of the formula (a), where $R^3$ is $C_1$-$C_3$-alkyl, $C_1$-$C_3$-alkoxy, chlorine or bromine, and $R^4$ is hydrogen, 4-hydroxy, 6-methyl, 6-methoxy, 6-fluoro or 6-chloro, and $R^6$ is hydrogen, and the physiologically tolerated salts thereof.

**4.** A compound as claimed in claim 3, where $R^1$ is methyl, methoxymethyl or hydroxymethyl, $R^2$ is 1-naphthyl or the group (a), where $R^3$ is methyl, ethyl, methoxy, chlorine or bromine, and $R^4$ is hydrogen or 6-fluoro or 6-chloro, $R^5$ is methoxy, methyl or ethyl, and $R^6$ is hydrogen, and the physiologically tolerated salts thereof.

**5.** A process for the preparation of a drug of the formula I

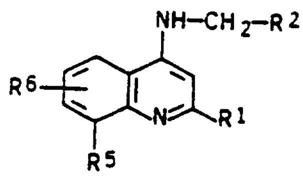

(I)

where

$R^1$ is $C_1$-$C_3$-alkyl which can be substituted by a hydroxyl or $C_1$-$C_3$-alkoxy group,

$R^2$ is either naphthyl or a radical of the formula (a)

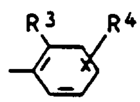

(a)

where

$R^3$ is $C_1$-$C_3$-alkyl which can be substituted by a hydroxyl or methoxy group, or is $C_1$-$C_3$-alkoxy, fluorine, chlorine or bromine, and

$R^4$ is hydrogen, methyl, hydroxyl, methoxy, fluorine, chlorine or bromine, and

$R^5$ is $C_1$-$C_3$-alkoxy, $C_1$-$C_3$-alkyl which can be substituted by a hydroxyl or $C_1$-$C_3$-alkoxy group, or is hydroxyl, fluorine, chlorine or bromine, and

$R^6$ is hydrogen, methyl, methoxy, fluorine, chlorine or bromine,

which comprises reacting a quinoline of the formula II

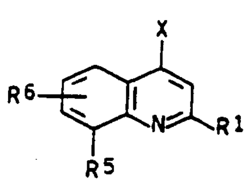

(II)

where X is a nucleophilic leaving group, and $R^1$, $R^5$ and $R^6$ have the meanings stated in claim 1, with

an amine of the formula $R^2CH_2NH_2$ in the presence or absence of a solvent at from 50 to 250 °C.

6. The use of a compound of the formula I as claimed in any of claims 1 to 4 for the preparation of a drug.

7. The use of a compound of the formula I as claimed in any of claims 1 to 4 for the preparation of a drug for disorders in which a reduction in gastric acid secretion has a beneficial effect on healing.

8. A drug for oral or rectal use, which contains as active compound 10 to 1000 mg of a compound of the formula I as claimed in any of claims 1 to 4 per single dose, besides conventional pharmaceutical auxiliaries.

9. A drug for intravenous administration, which contains as active compound 0.01 to 1 mg/kg of body weight of a compound of the formula I as claimed in any of claims 1 to 4, besides conventional pharmaceutical auxiliaries.

10. A drug for disorders in which a reduction in gastric acid secretion has a beneficial effect on healing, which contains as active compound an effective amount of a compound of the formula I as claimed in any of claims 1 to 4, besides conventional pharmaceutical auxiliaries.

## Revendications

1. 2-alkyl-4-arylméthylaminoquinoléines de formule générale I

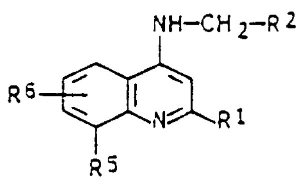

(I)

dans laquelle
$R^1$ représente un groupe alkyle en $C_1$-$C_3$ qui peut être substitué par un groupe hydroxy ou alcoxy en $C_1$-$C_3$,
$R^2$ représente un groupe naphtyle ou un groupe de formule a)

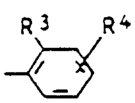

(a)

dans laquelle
$R^3$ représente un groupe alkyle en $C_1$-$C_3$ qui peut être substitué par un groupe hydroxy ou méthoxy, un groupe alcoxy en $C_1$-$C_3$, un atome de fluor, de chlore ou de brome et
$R^4$ représente un atome d'hydrogène, un groupe méthyle, hydroxy ou méthoxy, un atome de fluor, de chlore ou de brome, et
$R^5$ représente un groupe alcoxy en $C_1$-$C_3$, un groupe alkyle en $C_1$-$C_3$ qui peut être substitué par un groupe hydroxy ou alcoxy en $C_1$-$C_3$,un groupe hydroxy, un atome de fluor, de chlore ou de brome et
$R^6$ représente un atome d'hydrogène, un groupe méthyle, méthoxy, un atome de chlore ou de brome,
et leurs sels acceptables pour l'usage pharmaceutique.

2. Composé selon la revendication 1, pour lequel $R^5$ représente un groupe alcoxy en $C_1$-$C_2$, un groupe alkyle en $C_1$-$C_2$ qui peut être substitué par un groupe hydroxy ou méthoxy, et ses sels acceptables pour l'usage pharmaceutique.

3. Composé selon la revendication 2, pour lequel $R^1$ représente un groupe méthyle qui peut être substitué par un groupe hydroxy ou alcoxy en $C_1$-$C_3$, $R^2$ représente un groupe 1-naphtyle ou un groupe de formule (a) dans laquelle $R^3$ représente un groupe alkyle en $C_1$-$C_3$, un groupe alcoxy en $C_1$-$C_3$, un atome de chlore ou de brome et $R^4$ représente un atome d'hydrogène, un groupe 4-hydroxy, un groupe 6-méthyle, 6-méthoxy, un substituant 6-fluoro ou 6-chloro et $R^6$ représente un atome d'hydrogène, et ses sels acceptables pour l'usage pharmaceutique.

4. Composé selon la revendication 3, pour lequel $R^1$ représente un groupe méthyle, méthoxyméthyle ou hydroxyméthyle, $R^2$ représente un groupe 1-naphtyle ou le groupe (a) dans lequel $R^3$ représente un groupe méthyle, éthyle, méthoxy, un atome de chlore ou de brome et $R^4$ un atome d'hydrogène ou un substituant 6-fluoro ou 6-chloro, $R^5$ représente un groupe méthoxy, méthyle ou éthyle et $R^6$ un atome d'hydrogène, et ses sels acceptables pour l'usage pharmaceutique.

5. Procédé de préparation des composés de formule I

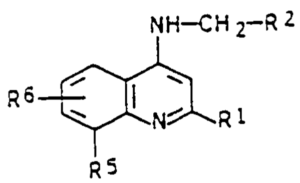

(I)

dans laquelle
$R^1$ représente un groupe alkyle en $C_1$-$C_3$ qui peut être substitué par un groupe hydroxy ou alcoxy en $C_1$-$C_3$, $R^2$ représente un groupe naphtyle ou un groupe de formule (a)

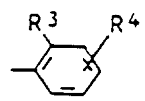

(a)

dans laquelle
$R^3$ représente un groupe alkyle en $C_1$-$C_3$ qui peut être substitué par un groupe hydroxy ou méthoxy, un groupe alcoxy en $C_1$-$C_3$, un atome de fluor, de chlore ou de brome et $R^4$ représente un atome d'hydrogène, un groupe méthyle, hydroxy ou méthoxy, un atome de fluor, de chlore ou de brome et
$R^5$ représente un groupe alcoxy en $C_1$-$C_3$, un groupe alkyle en $C_1$-$C_3$ qui peut être substitué par un groupe hydroxy ou alcoxy en $C_1$-$C_3$,un groupe hydroxy, un atome de fluor, de chlore ou de brome et
$R^6$ représente un atome d'hydrogène, un groupe méthyle, méthoxy, un atome de chlore ou de brome, caractérisé en ce que l'on fait réagir une quinoléine de formule II

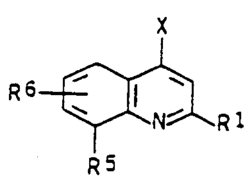

(II),

dans laquelle X représente un groupe éliminable nucléophile et $R^1$, $R^5$ et $R^6$ ont les significations indiquées dans la revendication 1, avec une amine de formule $R^2CH_2NH_2$ en présence ou en l'absence de solvant à une température dans l'intervalle de 50 à 250 °C.

6. Utilisation d'un composé de formule I selon l'une des revendications 1 à 4 pour la préparation d'un médicament.

7. Utilisation d'un composé de formule générale I selon une des revendications 1 à 4 pour la préparation d'un médicament contre les maladies pour lesquelles une diminution de la sécrétion d'acidité gastrique a une influence favorable sur la guérison.

8. Médicament pour l'administration orale ou rectale contenant en tant que substance active, et par dose individuelle, de 10 à 1000 mg d'un composé de formule I selon une des revendications 1 à 4, avec des produits auxiliaires galéniques usuels.

9. Médicament pour l'administration intraveineuse contenant en tant que substance active, en quantité de 0,01 à 1 mg par kg de poids corporel, un composé de formule I selon une des revendications 1 à 4 avec des produits auxiliaires galéniques usuels.

10. Médicament contre les maladies pour lesquelles une diminution de la sécrétion d'acidité gastrique a un effet favorable sur la guérison, et qui contient en tant que substance active une quantité efficace d'un composé de formule I selon l'une des revendications 1 à 4 avec des produits auxiliaires galéniques usuels.